# EUROPEAN PATENT APPLICATION

(11) **EP 4 649 944 A1**
(43) Date of publication of application: **19.11.2025**
(21) Application number: 25174150.0
(22) Date of filing: 05.05.2025
(51) Int. Cl.: A61K 9/20, A61K 31/5517

(54) **PHARMACEUTICAL COMPOSITION OF ALPRAZOLAM**

(30) Priority: 13.05.2024 CZ 20240189
(71) Applicant: Zentiva, K.S., 10200 Praha 10 - Dolni Mecholupy (CZ)
(72) Inventor: Kluk, Anna, Praha (CZ); Bartosi ska, Ewa, Praha (CZ)
(74) Representative: Harber IP s.r.o.

(57) **Abstract**

The present invention relates to a pharmaceutical composition comprising alprazolam, at least one pharmaceutically acceptable filler and at least one pharmaceutically acceptable disintegrant, wherein said pharmaceutically acceptable filler is CaCO₃ and/or microcrystalline cellulose, and said disintegrant is crospovidone. Such a composition has better stability of alprazolam and lower content of impurity G than commercially available compositions.

## Description

### Technical Field

Alprazolam in solid dosage form undergoes decomposition and the main impurity is triazolaminoquinoline, referred to as impurity G, which is genotoxic. The formation of impurity G is accelerated at higher storage temperatures and higher humidity. The present invention relates to a pharmaceutical composition of alprazolam, which is suitable for use in a solid dosage form, having an increased stability of said composition and reduced content of impurity G.

### Background Art

Alprazolam (8-chloro-1-methyl-6-phenyl-4*H*-s-triazolo-[4,3-a][1,4]-benzodiazepine; formula I) is an anxiolytic of the benzodiazepine class and is used as a medicament for anxiety suppression, sedation, disinhibition, and muscle relaxation. It binds to specific sites on the GABA_{A} receptor and is commonly used to treat panic disorder, generalized anxiety disorder (GAD), or social anxiety disorder (SAD).

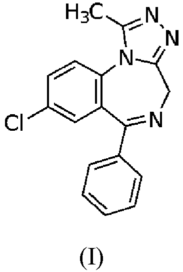

The article of A.L.Huidobro et al. (Journal of Pharmaceutical and Biomedicinal Analysis 44 (2007) 404-413*)* describes stability tests of alprazolam and the identification and isolation of its main degradation products (especially of impurity G; 7-chloro-1-methyl-5-phenyl-[1,2,4]triazolo[4,3-a]quinolin-4-amine; formula II).

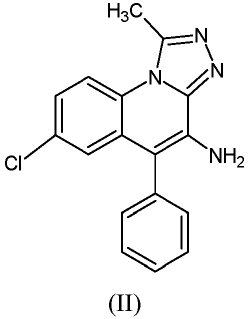

The authors state that temperature and humidity, even humidity originating from excipients present in the formulation, play a very important role in the formation of impurity G. On the contrary, its formation is independent of the presence of light.

Castaňeda et al. (*J. Phys. Org. Chem. 2009, 22, 807-814*) studied the stability of alprazolam and the influence of chemical interactions with some excipients of solid dosage forms on the formation of impurities, including impurity G, using spectrophotometry and HPLC methods. Alprazolam was studied in its pure form, which was surprisingly found to be very stable to hydrolysis, but is subject to photodegradation (> 400 days) to impurity G. The aforementioned impurity G surprisingly forms even in the absence of light when alprazolam is combined with various pharmaceutically acceptable excipients used in solid dosage forms. Therefore, the effect of four types of excipients on the stability of alprazolam (formation of impurity G after 8 and 15 hours at 80 °C and 80% RH) was investigated, namely the effect of diluent (lactose, corn starch), lubricant/glidant (magnesium stearate, talc), binder (polyvinylpyrrolidone) and disintegrant (sodium carboxymethylcellulose, CMC). The results showed that formulations with CMC and magnesium stearate are the least unstable. Reactions between the carboxyl functional groups present in both of these substances probably cause increased formation of impurity G. Therefore, for more stable solid formulations of alprazolam, excipients bearing carboxyl groups should not be used together with acidic or basic pH values in the formulation.

An analytical study of the degradation of alprazolam to impurity G by the Maillard reaction (a non-enzymatic reaction between a carbohydrate and an amino group) is described by A.L. Huidobro et al. in Anal Bioanal Chem (2009) 394: 1349-1359*.* The authors studied the degradation of alprazolam in combination with various excipients and observed that the conversion of alprazolam to impurity G is caused by: corn starch, microcrystalline cellulose (MCC), sodium carboxymethylcellulose (CMC) and especially lactose monohydrate; and they mention a probable synergistic effect of the above substances on the formation of impurity G. Replacing lactose (diluent) with non-reducing carbohydrates (mannitol, sorbitol) or calcium carbonate led to a significant reduction in the formation of impurity G.

The state of the art shows that, in particular, alprazolam formulations containing commonly used pharmaceutically acceptable excipients, such as starch derivatives, cellulose, magnesium stearate and/or lactose, are unstable and alprazolam in them degrades to impurity G. The aim of the present invention is therefore to prepare a pharmaceutical composition containing alprazolam, with sufficient stability in solid form to minimize the formation of genotoxic impurity G.

### Disclosure of Invention

The disclosed invention solves the problems of the prior art by providing a formulation of alprazolam with a specific combination of excipients, which increases the stability of the composition and prevents the formation of impurity G. The composition of the formulation can also prevent any subsequent degradation during storage.

A pharmaceutical composition is a composition of a drug/medicament for preparation into a dosage form, for example a mixture for tableting or for filling capsules.

An object of the present invention is a pharmaceutical composition comprising alprazolam as the active ingredient, and further comprising a mixture of pharmaceutically acceptable excipients, containing at least one pharmaceutically acceptable filler and at least one pharmaceutically acceptable disintegrant, wherein said pharmaceutically acceptable filler is CaCO₃ and/or microcrystalline cellulose (MCC) and said pharmaceutically acceptable disintegrant is crospovidone.

As shown in the examples below, the interaction of alprazolam and the combination of excipients plays a significant role in the stability of the pharmaceutical composition, since alprazolam, in presence of some excipients, undergoes ring opening for the Maillard reaction and subsequent conversion to the genotoxic impurity G.

The above combination of pharmaceutically acceptable excipients, however, surprisingly leads to higher stability of the pharmaceutical composition and to a significantly lower content of impurity G. The selection of this specific combination of the filler and the disintegrant, or rather its influence on the stability and content of impurity G, was not foreseeable from the state of the art. On the contrary, A.L.Huidobro et al. (Journal of Pharmaceutical and Biomedicinal Analysis 44 (2007) 404-413*)* attribute the influence on the formation of impurity G to temperature and humidity, not to a specific combination of excipients. Castaňeda et al. (*J. Phys. Org. Chem. 2009, 22, 807-814*) do not recommend the use of excipients bearing carboxyl groups together with acidic or basic pH values, in particular CMC and magnesium stearate. The authors A.L.Huidobro et al. in Anal Bioanal Chem (2009) 394: 1349-1359 even observed that the conversion of alprazolam to impurity G is caused, among others, by MCC.

In the present invention, the pharmaceutically acceptable filler is preferably a combination of CaCO₃ and microcrystalline cellulose (MCC), more preferably the weight ratio of CaCO₃ to microcrystalline cellulose is in the range of from 1:2 to 1:4, even more preferably in the range of from 1:2.5 to 1:3. Most preferably the weight ratio of CaCO₃ to microcrystalline cellulose is 1:3. Preferably, the filler content in the resulting pharmaceutical composition is at least 80 % by weight, more preferably the filler is present in the pharmaceutical composition in the amount of from 83 to 90 % by weight, most preferably in the amount of from 87 to 88 % by weight.

Preferably, the disintegrant content in the resulting pharmaceutical composition is at least 5 % by weight, more preferably from 5 to 15 % by weight, even more preferably from 8 to 12 % by weight, most preferably the content of the disintegrant is 10 % by weight.

Said pharmaceutical composition may contain further pharmaceutically acceptable excipients. In general, pharmaceutically acceptable excipients can be selected in particular from the group of fillers, binders, antioxidants, preservatives, surfactants, disintegrants, lubricants, glidants and substances affecting solubility.

Preferably, said further pharmaceutically acceptable excipient is selected from the group comprising fillers, binders, glidants, lubricants, surfactants, disintegrants, dyes, solvents, antimicrobials or olfactory and taste correctors commonly used in pharmacy in the manufacture of solid dosage forms.

More preferably, the filler is selected from the group comprising polysaccharides and their derivatives, in particular from the group of celluloses and their derivatives, for example microcrystalline cellulose, powdered cellulose, hydroxypropylmethyl cellulose, hydroxypropyl cellulose and combinations thereof.

According to the examples below, the glidants do not have any significant effect on the stability of alprazolam, and can therefore be selected from the group comprising silicon dioxide, talc and combinations thereof.

The lubricant can be selected from the group comprising magnesium stearate, calcium stearate and combinations thereof.

In a preferred embodiment, the content of alprazolam in the pharmaceutical composition is in the range of from 0.1 to 2 % by weight, preferably in the range of from 0.15 to 1.5 % by weight, more preferably in the range of from 0.18 to 1.1 % by weight.

In one embodiment, the pharmaceutical composition comprises:
- alprazolam as the active ingredient in the amount of from 0.1 to 2 % by weight;
- CaCO₃ as the filler in the amount of from 16 to 23 % by weight;
- MCC as the filler in the amount of from 65 to 67 % by weight;
- crospovidone as the disintegrant in the amount of from 5 to 15 % by weight;
- a glidant in the amount of from 0.2 to 0.3 % by weight; preferably the glidant is silicon dioxide;
- a lubricant in the amount of from 1 to 3 % by weight; preferably the lubricant is calcium stearate.

By replacing lactose as a filler with calcium carbonate and simultaneously replacing corn starch as a disintegrant with crospovidone lead surprisingly to significantly reduced the contamination of the composition with impurity G. On the contrary, the teaching of Castañeda et al. that excipients bearing carboxyl groups (e.g. stearate) have a negative effect on the stability of the composition was not confirmed, nor was the observation of the negative effect of MCC by A.L. Huidobro et al.

The above-described pharmaceutical composition of alprazolam is the most stable form of this active ingredient described to date and does not require any special handling precautions, its storage or packaging can remain the same as in the pharmaceutical compositions of alprazolam used to date. The pharmaceutical composition can be prepared in any solid dosage form, e.g. in the form of tablets, capsules, powders, pellets or granules. A preferred dosage form is a capsule, tablet or coated tablet. The capsule can be advantageously prepared by mixing alprazolam with pharmaceutically acceptable excipients and filling into the capsules after optional prior granulation. The tablet can be advantageously prepared by mixing alprazolam with pharmaceutically acceptable excipients and subjecting it to tableting after optional prior granulation, tableting may optionally be followed by coating.

The content of alprazolam in a unit solid dosage form is in the range of from 0.25 mg to 1 mg, preferably it may be 0.25 mg, 0.5 mg or 1 mg.

The pharmaceutical composition according to the invention has higher stability and better patient compliance than the commercially available compositions, because the genotoxic impurity G is not formed during storage.

### Brief Description of Drawings

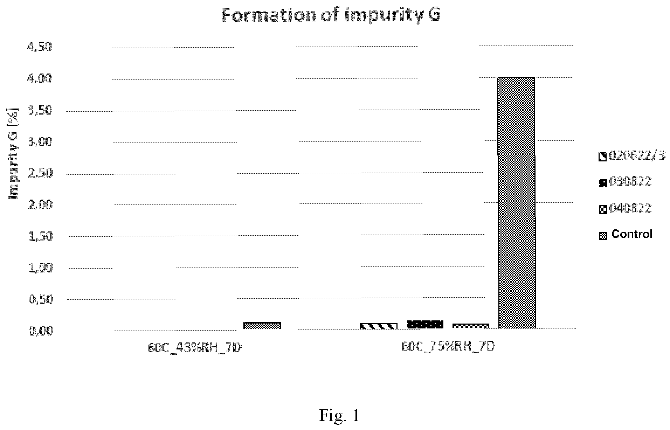

Fig. 1: Formation of impurity G of selected compositions at a temperature of 60 °C and humidities of 43% RH and 75% RH according to Example 4. A commercially used pharmaceutical composition of alprazolam containing lactose and MCC as fillers, corn starch as disintegrant, colloidal SiO₂ as glidant and calcium stearate as lubricant was used as a control.

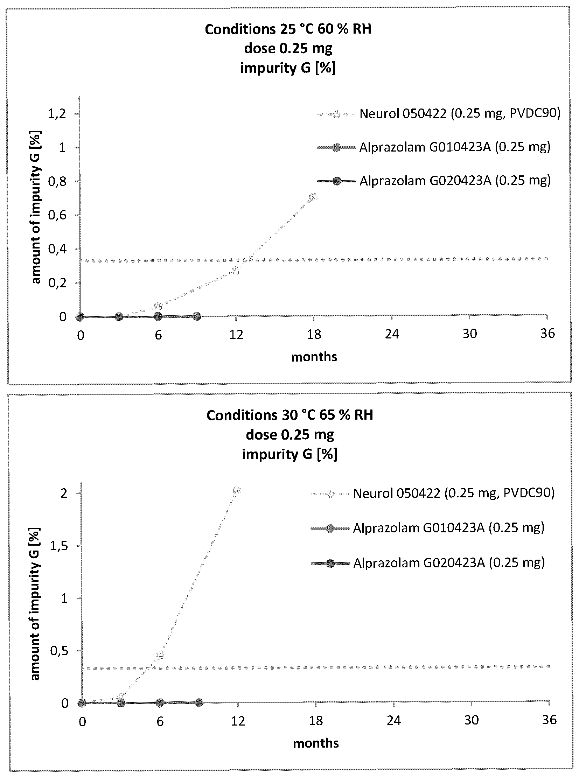

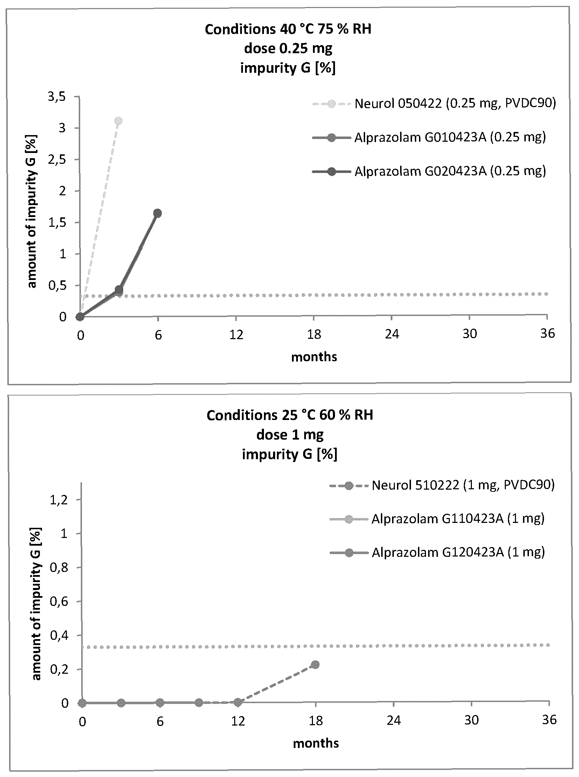

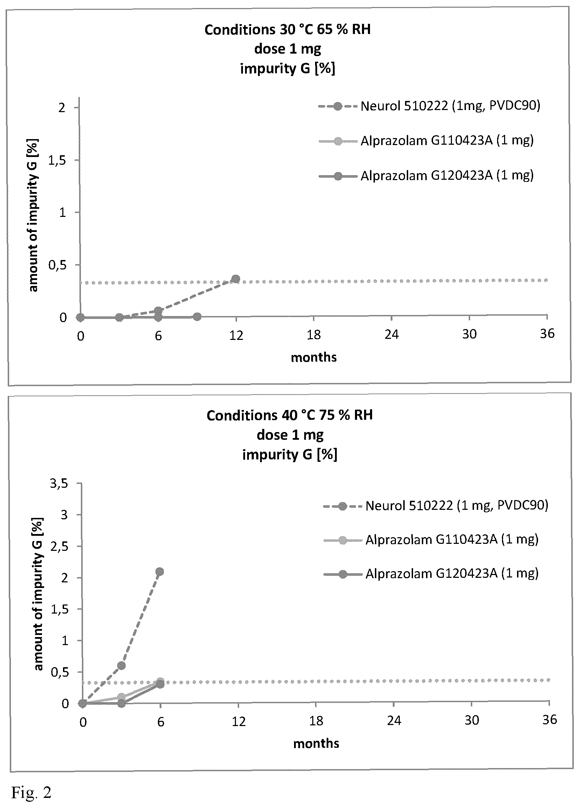

Fig. 2: Comparison of the amount of impurity G generated in standard stability tests in the compositions Neurol (composition according to the prior art) and Alprazolam (composition according to the invention) according to Example 5. The stability tests were carried out in open vials in which the tablets were exposed to given stress conditions (temperature, humidity) 25 °C/60%RH, 30 °C/65%RH and 40 °C/75%RH). The amount of impurity G was determined by HPLC with UV detection. The horizontal dotted line represents the limit value of impurity G according to EMA (European Medicines Agency).

### Examples

The following examples are provided for illustration and explanation of the invention only and are not intended to limit the scope of protection, which is limited only by the wording of the claims. All of the following experiments were performed with commercially available alprazolam.

### Identification of the mechanism of formation of impurity G (compatibility tests)

### Example 1: Compatibility test no. 1

Various excipients were tested and their effect on the stability of alprazolam was determined. For each of the excipients, an alternative excipient was identified, differing in pH value or in another property. It was found that, in accordance with J. Phys. Org. Chem. 2009, 22, 807-814*,* acidic conditions promote ring opening for the Maillard reaction (between API and lactose), but these conditions also promote interactions with other excipients. Therefore, acidic excipients (such as Ca(H₂PO₄)₂, stearic acid) should not be used in combination with alprazolam, especially not in combination with lactose. The test results are shown in Table 2.

The composition of the sample was set according to Table 1 below, wherein the individual excipients of the given function were changed according to Table 2. Alprazolam was sieved through a 0.63 mm sieve and mixed with the first part of filler 1, other components were sieved through a 1.00 mm sieve and everything was mixed again, followed by tableting. The tablets prepared in this way were placed in open bottles and exposed to stress conditions in a chamber at 60 °C/75 %RH for 4 days.

**Table 1: General composition of the sample**

| **composition** | | **mg/tbl** | **% w/w** |
|---|---|---|---|
| | Alprazolam | *1.00* | *0.71* |
| | Filler 1 (part 1) | *5.90* | *4.21* |
| | Filler 1 (part 2) | *17.70* | *12.64* |
| | Disintegrant | *20.55* | *14.68* |
| | Filler 2 | *91.65* | *65.46* |
| | Glidant | *0.40* | *0.29* |
| | Lubricant | *2.80* | *2.00* |
| Suma | | ***140.00*** | ***100.00*** |

**Table 2: Composition of tested alprazolam compositions; impurity G was determined by RP-HPLC (mobile phase: ammonium acetate, pH=4.2/methanol, according to gradient) with UV detection after 4 days at 60 °C and 75% RH. The pH of the tablets was not adjusted.**

| Exp. | Filler 1 | Filler 2 | Disintegrant | Glidant | Lubricant | Impurity G [%] | pH of tablets |
|---|---|---|---|---|---|---|---|
| 1 | Lactose | MCC | cornstarch | talc | stearic acid | 8.71 | 5.29 |
| 2 | Mannitol | MCC | cornstarch | colloidal silicon dioxide | calcium stearate | 0.97 | 6.4 |
| 3 | Lactose | Calc. hydr. phosph. | cornstarch | colloidal silicon dioxide | stearic acid | 12.18 | 5.3 |
| 4 | Mannitol | Calc. hydr. phosph. | cornstarch | talc | calcium stearate | 0.92 | 5.68 |
| 5 | Lactose | MCC | crospovidone | talc | calcium stearate | 0.46 | 6.35 |
| 6 | Mannitol | MCC | crospovidone | colloidal silicon dioxide | stearic acid | 0.91 | 6.01 |
| 7 | Lactose | Calc. hydr. phosph. | crospovidone | colloidal silicon dioxide | calcium stearate | 4.85 | 5.63 |
| 8 | Mannitol | Calc. hydr. phosph. | crospovidone | talc | stearic acid | 5.99 | 5.39 |
| 9 | Lactose | MCC | cornstarch | colloidal silicon dioxide | calcium stearate | 1.1 | 6.65 |
| 10 | Lactose | MCC | cornstarch | colloidal silicon dioxide | calcium stearate | 0.99 | 6.6 |
| 11 | Lactose | MCC | cornstarch | colloidal silicon dioxide | calcium stearate | 1.03 | 6.53 |

Lactose monohydrate, 350 mesh was used as lactose; MCC stands for microcrystalline cellulose PH 102; Pearlitol 50C was used as mannitol; Calc. hydr. phosph. stands for Ca(H₂PO₄)₂.

The best stability results were observed for formulation No. 5 (lactose, MCC, crospovidone, talc, calcium stearate). Both temperature and humidity promote the decomposition of alprazolam, therefore it is advisable to choose excipients containing less moisture.

The effect of light on the stability of alprazolam (photodegradation) was also studied. Although photodegradation of alprazolam was observed, the presence of impurity G was not detected as a result of exposure of alprazolam to UV radiation.

### Example 2: Compatibility test no. 2

It was found in Example 1 that MCC does not have any negative effect on the stability of alprazolam. A similar conclusion was made in relation to calcium stearate, which surprisingly supported the stability of alprazolam, probably due to its basic character. Therefore, these excipients were kept as a lubricant and a filler 2 in the subsequent tests and only the second filler (lactose and its potential alternatives mannitol and CaCO₃), disintegrant (corn starch or crospovidone) and glidant (silicon dioxide or talc) were varied. The content of individual components and their roles were the same as in the first compatibility test. The tablets were prepared in the same way as in Example 1.

The test results are shown in Table 3. Sample No. 15 contained lactose and CaCO₃ in a weight ratio of 1:1; samples No. 16-18 were prepared by different order of mixing of the excipients, wherein alprazolam was mixed with the first filler first, and then the other ingredients were added. The tablets were placed in open vials and exposed to stress conditions in a chamber of 40 °C/43%RH and 40 °C/75%RH.

**Table 3: The composition of excipients of the tested alprazolam compositions; impurity G was determined by RP-HPLC (mobile phase: ammonium acetate, pH=4.2/methanol, according to gradient) with UV detection after 27 days at 40 °C and 43 or 75% RH.**

| **Exp.** | **Filler 1** | **Disintegrant** | **Glidant** | **Impurity G 40°C/43% RV, 27D [%]** | **Impurity G 40°C/75% RV, 27D [%]** |
|---|---|---|---|---|---|
| 1 | Mannitol | cornstarch | colloidal silicon dioxide | <0.05 | 0.87 |
| 2 | Lactose | cornstarch | colloidal silicon dioxide | <0.05 | 0.86 |
| 3 | Calcium carbonate | crospovidone | colloidal silicon dioxide | <0.05 | <0.05 |
| 4 | Mannitol | crospovidone | colloidal silicon dioxide | <0.05 | 0.5 |
| 5 | Lactose | crospovidone | colloidal silicon dioxide | <0.05 | 0.41 |
| 6 | Calcium carbonate | cornstarch | talc | <0.05 | 0.37 |
| 7 | Mannitol | cornstarch | talc | <0.05 | 0.84 |
| 8 | Lactose | cornstarch | talc | <0.05 | 0.9 |
| 9 | Calcium carbonate | crospovidone | talc | <0.05 | <0.05 |
| 10 | Mannitol | crospovidone | talc | <0.05 | 0.49 |
| 11 | Lactose | crospovidone | talc | <0.05 | 0.36 |
| 12 | Calcium carbonate | cornstarch | colloidal silicon dioxide | <0.05 | 0.32 |
| 13 | Calcium carbonate | cornstarch | colloidal silicon dioxide | <0.05 | 0.32 |
| 14 | Calcium carbonate | cornstarch | colloidal silicon dioxide | <0.05 | 0.33 |
| 15 | Lactose + Calcium carbonate | cornstarch | colloidal silicon dioxide | <0.05 | 0.47 |
| 16 | Re-mixed with cornstarch | | | <0.05 | 0.91 |
| 17 | Re-mixed with MCC | | | <0.05 | 0.87 |
| 18 | Re-mixed with calcium carbonate | | | <0.05 | 0.89 |

The results show that crospovidone and CaCO₃ play a key role in the stability of alprazolam (as API) containing pharmaceutical composition, both at low and high humidity environments.

On the contrary, from other experiments (under stress conditions of 60 °C and 43%RH) it was observed that lactose promotes the degradation of alprazolam even in dry conditions. Mannitol as a substitute for lactose does not improve the stability of the API in any way, nor does the use of corn starch. Glidants do not show any significant effect on the stability of alprazolam.

Based on the above, compositions no. 3 and 9 were selected for further testing.

### Example 3: Qualitative composition - prediction of stability

Stability tests of alprazolam were performed with sample no. 3 from Example 2 at various temperatures (40 to 80 °C) and humidities (43 to 75% RH) in open vials, analogously to Examples 1 and 2. Compositions no. 12-14 were also tested for comparison and a commercially used pharmaceutical composition of alprazolam as a control. The composition of the tested samples is shown in Table 4 and the stability results are shown in Table 5.

**Table 4: Composition of the tested samples**

| Sample | API 0,71 % w/w | Filler 1 16,85 % w/w | Filler 2 65,46 % w/w | Disintegrant 14,68 % w/w | Glidant 0,29 % w/w | Lubricant 2 % w/w |
|---|---|---|---|---|---|---|
| 3 | Alprazolam | Calcium carbonate | MCC | crospovidone | colloidal silicon dioxide | calcium stearate |
| 12-14 | Alprazolam | Calcium carbonate | MCC | cornstarch | colloidal silicon dioxide | calcium stearate |
| kontr. | Alprazolam | Lactose | MCC | cornstarch | colloidal silicon dioxide | calcium stearate |

**Table 5: Results of chemical purity measurements of tested alprazolam compositions; impurity G was determined by RP-HPLC (mobile phase: ammonium acetate, pH=4.2/methanol, according to gradient) with UV detection.**

| **Time [days]** | **Tempeature [°C]** | **Humidity [%]** | **impurity G (sample 3) [%]** | **impurity G (sample 12-14) [%]** |
|---|---|---|---|---|
| **17** | **40** | **66** | <0.05 | 0.07 |
| **28** | **40** | **66** | <0.05 | 0.14 |
| **27** | **40** | **75** | <0.05 | 0.32 |
| **3** | **52** | **64** | <0.05 | <0.05 |
| **28** | **52** | **64** | 0.4 | 1.25 |
| **8** | **60** | **50** | 0.05 | 0.15 |
| **28** | **60** | **50** | 0.8 | 1.8 |
| **3** | **60** | **63** | <0.05 | 0.08 |
| **5** | **60** | **63** | 0.06 | 0.18 |
| **17** | **60** | **63** | 0.59 | 1.58 |
| **21** | **60** | **63** | 0.92 | 2.2 |
| **1** | **60** | **75** | <0.05 | <0.05 |
| **3** | **60** | **75** | <0.05 | 0.27 |
| **8** | **60** | **75** | 0.13 | 1.33 |
| **18** | **60** | **75** | 0.49 | 6.38 |
| **7** | **70** | **41** | <0.05 | 0.04 |
| **28** | **70** | **41** | 0.06 | 0.14 |
| **4** | **70** | **62** | 0.11 | 0.33 |
| **5** | **70** | **62** | 0.14 | 0.32 |
| **8** | **70** | **62** | 0.56 | 1.37 |
| **17** | **80** | **26** | 0.56 | 0.97 |
| **24** | **80** | **26** | 1.55 | 2.18 |
| **1** | **80** | **51** | <0.05 | 0.06 |
| **2** | **80** | **51** | 0.06 | 0.15 |
| **5** | **80** | **51** | 0.45 | 1.23 |
| **8** | **80** | **51** | 1.24 | 2.47 |
| **1** | **80** | **61** | 0.13 | 0.28 |
| **2** | **80** | **61** | 0.21 | 0.5 |
| **4** | **80** | **61** | 0.65 | 1.52 |

The experiment indicates that the composition no. 3 shows high stability. The other compositions are less stable, probably due to the moisture content, which contributes to the degradation of alprazolam. When compared to the commercially used pharmaceutical composition (its content corresponds to the sample no. 2 in Table 3), the tested samples according to the present invention were significantly more stable. The commercial sample had worse results (0.86 % imp. G) than samples no. 3 and 12-14 (row 3 in Table 5) under conditions of 40/75 already after 27 days.

### Example 4: New pharmaceutical composition, stability tests

Four compositions were prepared according to Table 6. The compositions were tested for content uniformity and dissolution properties. Stability tests were performed at 60 °C and humidity of 43 and 75% RH in open vials, analogously to Examples 1, 2 and 3. The stability tests of all three new compositions 020622/3, 040822 and 030822 were better than that of the commercial pharmaceutical composition (control) known from the prior art, see Fig. 1.

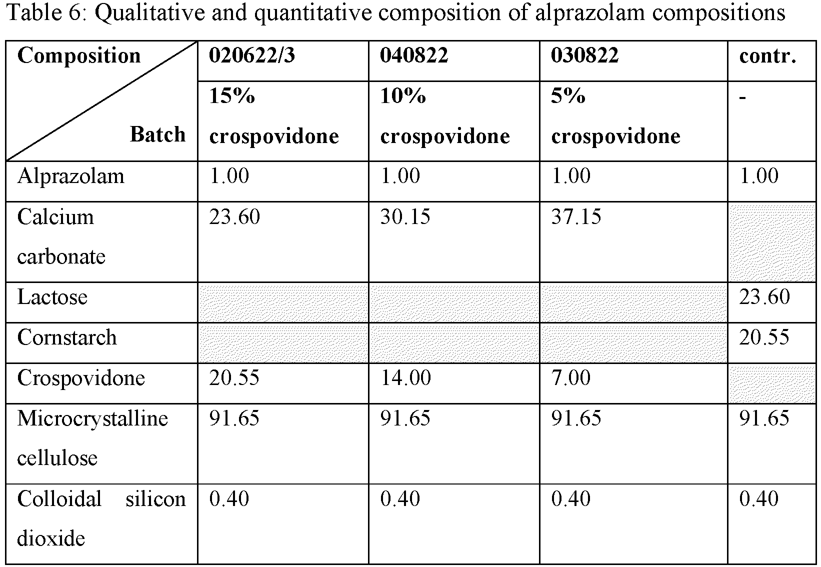

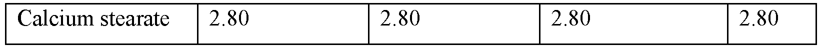

The reason for testing the reduction of crospovidone content was to prevent potential softening of the tablets due to swelling of crospovidone.

Impurity G was only formed at very high temperatures and humidity (Fig. 1). All three tested compositions were significantly more stable than the control sample (the prior art composition). The appearance of alprazolam tablets with the tested formulation was monitored after 3 and 6 months of storage at 25°C and 60% RH and no changes were observed.

### Example 5: Examples of new alprazolam tablet compositions

Based on the composition with the best chemical purity in terms of the formation of impurity G (see Examples above), compositions for tablets with a dose of 1 mg and 0.25 mg were designed and compared with compositions according to the state of the art. The AP content is higher in both cases, assuming a technological surplus due to production losses. The tested compositions are given in Table 7. Significantly worse stability of the compositions, due to the presence of impurity G, was observed for compositions according to the state of the art (compositions "Neurol", see Fig. 2).

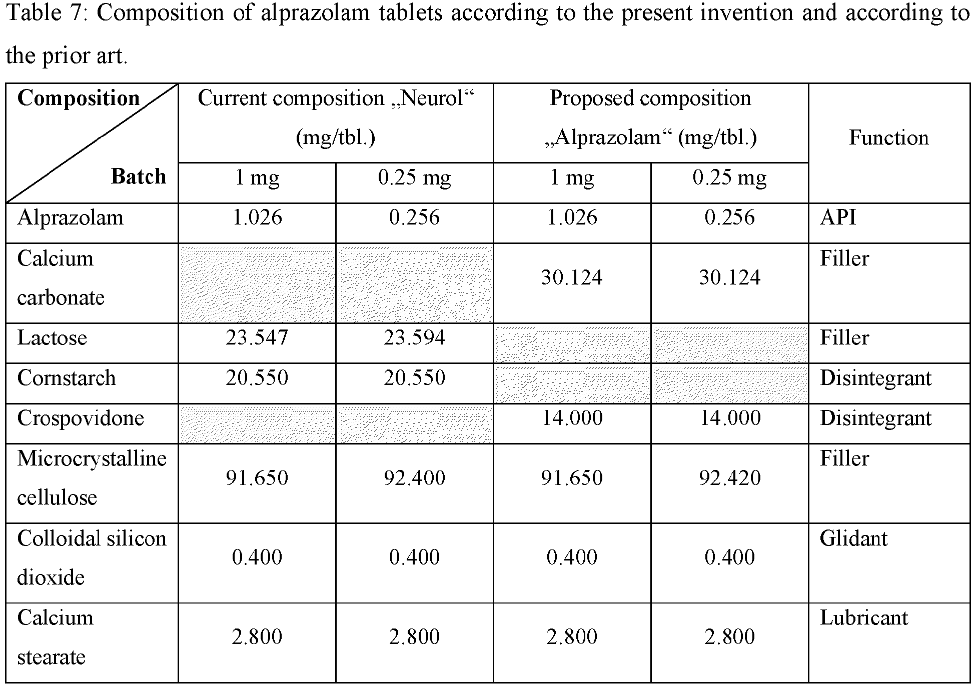

Both new compositions were manufactured in quantities of 140 kg (1,000,000 tablets) and showed higher stability with respect to the formation of impurity G compared to Alprazolam tablets of the same strength from the prior art. The graphs in Fig. 2 illustrate the stability (amount of impurity G) of the current and proposed Alprazolam tablet compositions in doses of 1 mg and 0.25 mg, packaged in PVDC90 blisters. Stability tests were carried out analogously to Example 2, the tablets were placed in open vials and exposed to stress conditions in a chamber (25 °C/60%RH, 30 °C/65%RH and 40 °C/75%RH).

## Claims

1. A pharmaceutical composition comprising alprazolam as an active ingredient, which further comprises a mixture of excipients containing at least one pharmaceutically acceptable filler and at least one pharmaceutically acceptable disintegrant,
**characterized in that**
said pharmaceutically acceptable filler is CaCO₃ and/or microcrystalline cellulose, and said disintegrant is crospovidone.

2. The pharmaceutical composition according to claim 1, which contains CaCO₃ and microcrystalline cellulose as pharmaceutically acceptable fillers.

3. The pharmaceutical composition according to claim 1 or 2, wherein said mixture of excipients further comprises a pharmaceutically acceptable glidant selected from silicon dioxide and/or talc.

4. The pharmaceutical composition according to claim 1, 2 or 3, wherein said mixture of excipients further comprises a pharmaceutically acceptable lubricant, selected from calcium stearate and/or magnesium stearate.

5. The pharmaceutical composition according to claim 2, 3 or 4, wherein the weight ratio of CaCO₃ to microcrystalline cellulose is in the range of from 1:2 to 1:4, preferably in the range of from 1:3 to 1:3,5.

6. The pharmaceutical composition according to any one of the preceding claims 1 to 5, wherein the content of the disintegrant is in the range of from 5 to 15 % (w/w), preferably in the range of from 8 to 12 % (w/w).

7. The pharmaceutical composition according to any one of the preceding claims 1 to 6, wherein the content of alprazolam in said pharmaceutical composition is in the range of from 0,1 to 2 % (w/w), preferably in the range of from 0,15 to 1,5 % (w/w), more preferably in the range of from 0,18 to 1,1 % (w/w).

8. The pharmaceutical composition according to any one of the preceding claims 1 to 7, comprising alprazolam as an active ingredient, and a mixture of excipients comprising a filler, a disintegrant, a glidant and a lubricant, wherein:
- the content of alprazolam in the pharmaceutical composition is in the range of from 0,1 to 2 % (w/w);
- the filler is CaCO₃ and microcrystalline cellulose, wherein the content of CaCO₃ in the pharmaceutical composition is in the range of from 16 to 23 % (w/w) and the content of microcrystalline cellulose in the pharmaceutical composition is in the range of from 65 to 67 % (w/w);
- the disintegrant is crospovidone, and its content in the pharmaceutical composition is in the range of from 5 to 15 % (w/w);
- the glidant is silicon dioxide, and its content in the pharmaceutical composition is in the range of from 0,2 to 0,3 % (w/w);
- the lubricant is calcium stearate, and its content in the pharmaceutical composition is in the range of from 1 to 3 % (w/w).

9. The pharmaceutical composition according to any one of the preceding claims 1 to 8, which is in a solid dosage form, preferably in the form of tablets, coated tablets or capsules.
